Europäisches Patentamt

European Patent Office

Office européen des brevets

(11) Publication number: **0 461 802 A2**

# EUROPEAN PATENT APPLICATION

(21) Application number: 91305069.6

(22) Date of filing: 05.06.91

(51) Int. Cl.5: **B01J 21/18**, B01J 37/08,
C07C 51/12, C07C 53/08

(30) Priority: 12.06.90 GB 9013116

(43) Date of publication of application:
18.12.91 Bulletin 91/51

(84) Designated Contracting States:
AT BE CH DE DK ES FR GB IT LI LU NL SE

(71) Applicant: THE BRITISH PETROLEUM
COMPANY P.L.C.
Britannic House, 1 Finsbury Circus
London EC2M 7BA(GB)

(72) Inventor: Lear, Anthony Martin, The British
Petroleum Co.
p.l.c., Chertsey Road, Sunbury-on-Thames
Middlesex, TW16 7LN(GB)
Inventor: Rawlinson, Anthony Paul
17 Croysdale Avenue, Sunbury-on-Thames
Middlesex, TW16 6QP(GB)
Inventor: Price, Peter John
Königswinterer Strasse 269
W-5300 Bonn 3(DE)

(74) Representative: Ryan, Edward Terrence et al
BP INTERNATIONAL LIMITED Patents &
Agreements Division Chertsey Road
Sunbury-on-Thames Middlesex, TW16
7LN(GB)

(54) Carbonylation of methanol.

(57) An alcohol is carbonylated with carbon monoxide over a carbonylation catalyst supported on a carbon derived from phenolic resin which has been partially cured, ground, shaped, carbonised, and activated by heating with alkali metal hydroxide and/or heating in an oxidising atmosphere.

EP 0 461 802 A2

The present invention relates to the carbonylation of methanol and to catalysts suitable for use in this reaction.

The carbonylation of methanol, i.e. the reaction of methanol with carbon monoxide and hydrogen, has been known for many years. Originally the reaction was carried out using homogeneous catalysis with the catalyst dissolved in a liquid reaction medium. More recently catalysts supported on solid carriers have been disclosed. Thus K K Robinson et al, Catalysis, 27, 389-986 (1972) disclose the vapour phase carbonylation of methanol to acetic acid using as catalyst rhodium on an activated carbon support. K Fujimoto et al, Ind. Eng, Chem. Prod. Res, Dev, 1983, 22, 436-439 disclose the carbonylation of methanol in the vapour phase over a catalyst comprising nickel on an active carbon support.

The unpublished pending European patent specification EP 0 335 625A describes a process for the production of a carboxylic acid from an alcohol using a catalyst comprising rhodium and nickel on a carbon support in the presence of a halogen or halogen compound promoter.

The specific carbon exemplified in this specificationn is an active carbon derived from natural sources such as coconut shell. Similar carbons are used in other references mentioned above.

EP 0 254 551A discloses a method of making shaped porous carbon articles from phenolic resin precursors. One use mentioned for these shaped carbon articles is as catalyst supports. However, no examples are given of the use of the carbon in catalyst production.

It would be desirable to produce a carbonylation catalyst of increased strength that could be made in a variety of physical forms but which still gave a high overall conversion of alcohols such as methanol to products. The initially formed carboxylic acid may react with the alcohol to give the ester. Where the object is to produce carboxylic acid then it is desirable to have a high selectivity to carboxylic acid in the final product.

It has now been found that improved catalysts may be obtained by selecting an appropriate carbon support.

According to the present invention the process for making a shaped porous carbon suitable for use as a support for a carbonylation catalyst is characterised by:
(a) partially curing a phenolic resin mixture to a solid,
(b) comminuting the partially cured resin,
(c) forming the comminuted resin to a pre-determined shape,
(d) sintering the shaped comminuted resin so as to produce a form-stable sintered product,
(e) carbonising the shaped sintered product, the duration and the temperature of the curing step (b) being so selected as to give a degree of cure sufficient to give a sinterable product, and
(f) activating the carbon by heating it
   (i) with alkali metal hydroxide, and/or
   (ii) by heating in an oxidising atmosphere.

According to another aspect of the present invention a catalyst suitable for use in the carbonylation of alcohols with carbon monoxide and hydrogen and which comprises a metal active for carbonylation on a carbon support is characterised in that the carbon is prepared by the steps of:
(a) partially curing a phenolic resin mixture to a solid,
(b) comminuting the partially cured resin,
(c) forming the comminuted resin to a pre-determined shape,
(d) sintering the shaped comminuted resin so as to produce a form-stable sintered product,
(e) carbonising the shaped sintered product, the duration and temperature of the curing step (b) being so selected as to give a degree of cure sufficient to give a sinterable product, and
(f) activating the carbon
   (i) by heating it with alkali metal hydroxide, and/or
   (ii) by heating in an oxidising atmosphere.

According to a further aspect of the present invention the process for the carbonylation of an alcohol with carbon monoxide over a catalytic metal on a carbon support is characterised in that the carbon is prepared by the successive steps of:
(a) partially curing a phenolic resin mixture to a solid,
(b) comminuting the partially cured resin,
(c) forming the comminuted resin to a pre-determined shape,
(d) sintering the shaped comminuted resin so as to produce a form-stable sintered product,
(e) carbonising the shaped sintered product, the duration and the temperature of the curing step (b) being so selected as to give a degree of cure sufficient to give a sinterable product, and
(f) activating the carbon
   (i) by heating it with alkali metal hydroxide, and/or

2

(ii) by heating with an oxidising gas.

The initial steps for the manufacture of the carbon catalyst support correspond to those described in EP 0 254 551.

The additional activation step with alkali metal hydroxide may be carried out by mixing the carbon with 50 to 500% by weight of granular alkali metal hydroxide. The alkali metal hydroxide may be a single alkali metal hydroxide or may be a mixture of alkali metal hydroxides. Preferably KOH or a mixture of KOH and NaOH is used. The weight percentages of alkali metal hydroxide are based on the weight of commercial quality material used, which may contain water (up to 20%).

After the carbon and alkali metal hydroxide have been mixed the mixture is heated to melt the alkali metal hydroxides and agitated to ensure that the carbon is wetted by the molten hydroxide. The mixture is then heated, preferably to temperatures in the range 700°C to 1000°C. The mixture is then allowed to cool and washed with water to remove residual alkali metal hydroxide.

The activation step may also be a treatment with a reactive oxygen-containing gas at elevated temperatures. The gas may contain molecular oxygen and may be air. Alternatively gases containing combined oxygen. e.g. carbon dioxide or steam may be used.

If gases containing molecular oxygen are used then temperatures in the range 250°-650°C are preferably used. The treatment with reactive gas is preferably carried out so as to produce a weight loss in the range 5-60% of the carbon using heating times in the range 0.5 to 24 hours.

If carbon dioxide is used for activation then temperatures in the range 700°-1200°C are preferably used.

Examples of metals which may be used in carbonylation catalysts of the present invention are rhodium, nickel, cobalt, or iridium.

The catalyst may be prepared by impregnating the carbon support with a solution or solutions of rhodium, nickel, cobalt, or iridium compounds. The solutions are most conveniently aqueous solutions, and the metal compounds are preferably water-soluble salts.

After the metal compounds have been deposited the solvent is removed. The impregnated carbon is then subjected to reduction in a reducing gas, e.g. hydrogen, at elevated temperatures, e.g. 100°-300°C, typically about 400°C to give the active catalyst.

The carbonylation process involves bringing an alcohol, e.g. methanol, and a gas comprising carbon monoxide, e.g. a mixture of carbon monoxide and hydrogen, into contact with the catalyst at elevated temperature and pressure. Examples of suitable temperatures are those in the range from 150° to 300°C and pressures in the range 1 to 50 atmospheres (gauge) (0.2 to 5 MPa).

The process is preferably carried out in the presence of a halogen or halogen compound as a promoter. Examples of suitable promoters are hydrogen halides, alkyl halides, or ammonium or phosphonium halides. The preferred halogen is iodine, preferably in the form of methyl iodide.

The process may be operated batchwise or continuously using fixed or moving beds. The LHSV (liquid hourly space velocity) for continuous operation is preferably in the range 0.1 to 5. The gas to liquid vapour ratio is preferably in the range 1:5 to 100:1. For gas mixtures the $CO:H_2$ volume ratio should be in the range from 0.05:1 to 20:1. It is particularly preferred to use volume ratios in the range 0.5:1 to 2:1.

The invention will now be described with reference to the following experiments in which examples of the invention are identified by numbers and comparative tests, not according to the invention, are identified by letters.

Example 1

This example describes the preparation of a carbon support in accordance with the invention, which has been activated by heating with KOH.

The carbon was prepared as follows.

The resin used in the following examples was finely ground phenol formaldehyde novolak resin containing 2.5 to 3% hexamine. This material was supplied by BP Chemicals under the designation J11011/1 (subsequently called J10 5OH). The resin particle size is 98 vol % less than 75 micrometres. The resin powder was poured into trays and cured for 2 hours at 150°C. The resulting slabs of partially but not completely cured resin were broken up into lumps and ground using a hammer mill which produced a particle size of 90 vol % below 500 micrometres.

The hammer milled material was ground to a fine particle size using a rotor and screen mill (blast mill) manufacturer by Condux. The resulting material had 90% of its volume between 11 and 107 micrometres (mean particle size 38 micrometres). 1.5 kg of this material was mixed with a solution of 120 g hexamine and 200 g polyethylene glycol in 500 ml distilled water in a Z-blade mixer. The dough produced was front

3

extruded through a die plate containing a large number of 2 mm diameter holes using a Fuji Paudal EXDFS 60 extruder. The resulting extrudate was sintered and carbonised by heating in nitrogen to 150°C at 5°/min, holding for 30 minutes and heating to 900°C at 5°/min and holding for 30 minutes. This carbon was heated to 1000°C in helium using an Astro graphite element furnace. Activation of the carbon was carried out by placing the material in a nickel crucible with its own weight of KOH pellets, heating the pellets in a nitrogen atmosphere to melt them, stirring the molten KOH and the carbon extruded pellets together, and then heating the mixture to a maximum temperature of 850°C, where it was held for 1.5 hours. The mixture was then allowed to cool to room temperature. It was then boiled for 10 minutes with distilled water, then with dilute hydrochloric acid, and again with distilled water, to remove KOH residues.

Example 2

This example shows the preparation of a catalyst from the carbon of Example 1 and its use to catalyse the reaction of methanol with carbon monoxide and hydrogen.

The carbon was sieved to give a fraction with a particle size in the range 500 to 1000 micrometres.

A sample of the carbon made in Example 1 was brought into contact with an aqueous solution containing the required amounts of rhodium and nickel nitrates.

The carbon was allowed to stand for 1 hour. Water was then removed by subjecting the impregnated carbon to a reduced pressure of 800 mbar (0.08 MPa) at a temperature of 60°C. The impregnated carbon was then reduced by passing hydrogen over it at 290°C for 3 hours.

The catalyst contained 1% weight rhodium and 2.5% by weight of nickel based on total weight of carbon.

The catalyst (2 ml) was placed in a reactor through which a gaseous feed was passed at a pressure of 145 psig (1.1 MPa absolute) while maintaining the temperature in the range given in the table. The gaseous feed consisted of hydrogen, carbon monoxide, methanol, and methyl iodide in the molar ratio 66:33:19:1. The methyl iodide acted as a promoter. The hydrogen and carbon monoxide were fed at the rates of 59.2 ml/min and 29.6 ml/min (RTP) (room temperature and pressure). The methanol and methyl iodide were introduced into the mixture of carbon monoxide and hydrogen as a liquid mixture at a total rate of 0.032 ml/min. The gas leaving the reactor was analysed and the overall methanol conversion and the overall selectivities to methane, acetic acid, and methyl acetate were determined. The results obtained during a 22 hour steady state period at a temperature of 210-215°C are given in the table under Example 2(i). The results for a steady state period of 15 hours and a temperature of 205°-209°C are given in the table under Example 2(ii).

Example 3

This shows the preparation of a carbon support by a process including heat treatment with an oxidising gas after activation with KOH.

The carbon from Example 1 was spread in a silica tray and placed in an air oven at 320°C. The temperature was raised in steps to 420°C over a period of 6.5 hours. After this time the carbon had lost 20.7% of its original weight.

Example 4

A catalyst was prepared and tested as in Example 2 but using the carbon of Example 3 as the support. The results obtained during a 26 hour steady state period are shown in the Table.

Example 5

A catalyst was prepared and tested as in Example 4.

The results obtained at a temperature of 205°-209°C for a steady state period of 17 hours and for a subsequent steady state period of 8 hours at a temperature of 195°-205°C are shown in the Table under 5-(i) and 5(ii) respectively.

Comparative Test A

This is a comparative test not according to the invention. A catalyst was prepared as in Example 2, except that the carbon used as support was a commercially available granular active carbon derived from coconut Shell and available from the firm of Sutcliffe Speakman as AC 610. It was used to carbonylate methanol as in Example 3 except that the composition of the product gases was determined after 14 hours steady state operation. The results are given in the table.

Comparative Test B

Methanol carbonylation as in Example 2 was carried out using a catalyst similar to that used in Comparative Test A, but in which the carbon had been pre-washed in $HNO_3$. Results obtained during a 13 hour steady state period are given in the Table.

Comparative Test C

An experiment was carried out as in Comparative Test A except that the carbon used was a commercially available active carbon extrudate derived from pine wood available from the firm of CECA. The results were measured over a 13 hour steady period starting 2 hours after coming on stream.

Comparative Test D

A catalyst was prepared and tested as in Example 2 using the phenolic resin carbon which had not been subjected to any treatment, such as activation by KOH or oxidation with air, after the carbonisation step. The results are given in the Table.

TABLE 1

| Experiment | Temp °C | Overall $CH_3OH$ conversion% | Overall selectivity % | | |
|---|---|---|---|---|---|
| | | | $CH_4$ | $CH_3COOH$ | $CH_3COOCH_3$ |
| A | 200-205 | 79.4 | 7.2 | 7.6 | 85.7 |
| B | 207 | 98.5 | 6.5 | 72.5 | 20.8 |
| C | 210-225 | 74.7 | 6.9 | 1.1 | 91.9 |
| D | 204 | 13.0 | 15.4 | 20.0 | 23.6 |
| 2(i) | 210-215 | 98.4 | 12.6 | 58.0 | 25.1 |
| 2(ii) | 205-209 | 87.3 | 5.2 | 18.8 | 69.7 |
| 4 | 206-210 | 65.0 | 2.8 | 8.4 | 86.3 |
| 5(i) | 205-209 | 77.4 | 2.7 | 18.4 | 77.2 |
| 5(ii) | 195-205 | 86.2 | 4.1 | 27.8 | 67.3 |

The crush strength of the carbon prepared in Example 3 was 0.51 kg/mm. The crush strength of the carbon made in Comparative Test C was only 0.35 kg/mm. The crush strength was measured as in EP 254 551.

The results given in the table show that replacing the activated carbon supports used in methanol carbonylation catalysts with a porous carbon made from a phenolic resin in accordance with EP 0 254 551 gives a catastrophic fall in catalyst activity as can be seen from a comparison of Test D with Tests A and B. However, when using carbon supports which have been subjected to additional treatment steps in accordance with the present invention the activity of the catalyst is greatly increased.

Example 6

5

The carbon from Example 1 was heat treated in helium at a rate of 50°C/minute to 1440°C and held for 30 minutes.

The resulting material was heated in a tube furnace in flowing $CO_2$ to 1000°C and held for 30 minutes. After this time the carbon had lost 39% of its weight. Results are given in Table 2 for a temperature of 211 to 221°C under Example 6(i) and for a subsequent period at a temperature of 199 to 201°C under Example 6(ii).

TABLE 2

| Experiment | Temp °C | Overall $CH_3OH$ conversion% | Overall selectivity % | | |
|---|---|---|---|---|---|
| | | | $CH_4$ | $CH_3COOH$ | $CH_3COOCH_3$ |
| 6(i) | 211-221 | 96.9 | 13.7 | 45.9 | 35.5 |
| 6(ii) | 199-201 | 85.7 | 5.4 | 13.1 | 77.4 |

**Claims**

1. A process for making shaped porous carbon suitable for use as a support for a carbonylation catalyst is characterised by:
   (a) partially curing a phenolic resin mixture to a solid,
   (b) comminuting the partially cured resin,
   (c) forming the comminuted resin to a pre-determined shape,
   (d) sintering the shaped comminuted resin so as to produce a form-stable sintered product,
   (e) carbonising the shaped sintered product, the duration and the temperature of the curing step (b) being so selected as to give a degree of cure sufficing to give a sinterable product, and
   (f) activating the carbon by heating it
      (i) with alkali metal hydroxide, and/or
      (ii) by heating in an oxidising atmosphere.

2. A process according to claim 1 wherein the activation with alkali metal hydroxide is carried out by mixing the carbon with 50 to 500% by weight of alkali metal hydroxide particles, melting the hydroxide, agitating the mixture to wet the carbon with molten hydroxide, and then heating to a temperature in the range 700°C to 1200°C.

3. A process according to either one of the preceding claims wherein the activation step comprises heating the carbon with a gas containing molecular oxygen at a temperature 250-650°C.

4. A process according to either one of claims 1 or 2 wherein the activation step is carried out by heating with carbon dioxide at a temperature in the range 700-1200°C.

5. A process according to either one of claims 3 or 4 wherein the activation step is carried out so as to produce a weight loss of 5-60% of the carbon.

6. A catalyst suitable for use in the carbonylation of alcohols with carbon monoxide and hydrogen and which comprises a metal active for carbonylation on a carbon support is characterised in that the carbon is prepared by the steps of:
   (a) partially curing a phenolic resin mixture to a solid,
   (b) comminuting the partially cured resin,
   (c) forming the comminuted resin to a pre-determined shape,
   (d) sintering the shaped comminuted resin so as to produce a form-stable sintered product,

(e) carbonising the shaped sintered product, the duration and the temperature of the curing step (b) being so selected as to give a degree of cure sufficient to give a sinterable product, and

(f) activating the carbon
  (i) by heating it with alkali metal hydroxide, and/or
  (ii) by heating in an oxidising atmosphere.

7. A catalyst according to claim 6 wherein the metal is rhodium, nickel, cobalt, or iridium.

8. A process for the carbonylation of an alcohol with carbon monoxide over a catalytic metal on a carbon support is characterised in that the carbon is prepared by the successive steps of:
  (a) partially curing a phenolic resin mixture to a solid,
  (b) comminuting the partially cured resin,
  (c) forming the comminuted resin to a pre-determined shape,
  (d) sintering the shaped comminuted resin so as to produce a form-stable sintered product,
  (e) carbonising the shaped sintered product, the duration and the temperature of the curing step (b) being so selected as to give a degree of cure sufficient to give a sinterable product, and
  (f) activating the carbon
    (i) by heating it with alkali metal hydroxide, and/or
    (ii) by heating with an oxidising gas.

9. A process according to claim 8 carried out at a temperature of $150°$-$300°$C, and pressures in the range 0.2 to 5 MPa (absolute).

10. A process according to claims 8 or 9 carried out in the presence of a halogen or halogen compound as promoter.

11. A process according to any one of claims 8 to 10 carried out continuously at an LHSV of 0.1 to 5, a gas to liquid volume ratio of 1:5 to 100:1 and a $CO:H_2$ volume ratio in the range 0.1:1 to 2:1.